# EUROPEAN PATENT APPLICATION

(11) **EP 3 939 630 A1**
(43) Date of publication of application: **19.01.2022**
(21) Application number: 20769569.3
(22) Date of filing: 07.02.2020
(51) Int. Cl.: A61M 1/02, A61J 1/05

(54) **BLOOD BAG SYSTEM**

(30) Priority: 14.03.2019 JP 2019047068
(71) Applicant: TERUMO Kabushiki Kaisha, Tokyo 151-0072 (JP)
(72) Inventor: AGEISHI, Aya, Tokyo 163-1450 (JP); MARUTA, Chiaki, Tokyo 163-1450 (JP)
(74) Representative: TBK
(86) International application number: PCT/JP2020/004798
(87) International publication number: WO 2020/184020

(57) **Abstract**

A blood bag system (10) includes a PPP bag (24) and a liquid drug bag (26) which are accommodated adjacent to each other in pockets (57) of a centrifugal drum (48) of a centrifugal transport device (12). In addition, the blood bag system (10) includes a retaining portion (94) that retains the PPP bag (24) and the liquid drug bag (26) together, and includes a cassette (84) accommodated in the pockets (57) in a retaining state of the retaining portion (94). The cassette (84) has a detachment mechanism (95) capable of detaching at least the PPP bag (24).

## Description

### Technical Field

The present invention relates to a blood bag system set in a centrifugal transport device.

### Background Art

A blood bag system including a blood bag storing blood, a PPP bag storing platelet poor plasma, which is a blood component, a liquid drug bag storing a red blood cell preservation solution, and the like is set in a centrifugal transport device at the time of centrifuging blood (see JP H5-184667 A). The centrifugal transport device applies a centrifugal force to the set blood bag to separate the blood in the blood bag into a plurality of types of blood components, and transports a plasma component to the PPP bag via a tube connected to the blood bag. After taking out the blood bag system from the centrifugal transport device, the liquid drug in the liquid drug bag is transported to the blood bag in which concentrated red blood cells remain.

### Summary of Invention

Meanwhile, when the blood bag system is set, the PPP bag is accommodated in a PPP bag pocket (bag accommodating portion) of the centrifugal transport device, and the liquid drug bag is accommodated in a liquid drug bag pocket (bag accommodating portion) of the centrifugal transport device. At this time, a tube connected to the PPP bag or the liquid drug bag is also accommodated in a predetermined path of the centrifugal transport device or the liquid drug bag pocket. If the tube projects, the tube is likely to interfere with a peripheral structure of the centrifugal transport device and break during centrifugation.

However, the work of accommodating the PPP bag and the liquid drug bag in the respective pockets while disposing the tube in the predetermined path requires a large amount of time and effort for a user, and there is a problem that it takes time to set the blood bag system with respect to the centrifugal transport device.

The present invention has been made to solve the above problem, and an object thereof is to provide a blood bag system capable of significantly improving working efficiency by facilitating work of disposing a plurality of bags in a centrifugal transport device with a simple configuration.

In order to achieve the above object, an aspect of the present invention is a blood bag system including: a first bag and a second bag which are accommodated adjacent to each other in a bag accommodating portion of a centrifugal drum of a centrifugal transport device; and a cassette which has a retaining portion that retains both the first bag and the second bag and is accommodated in the bag accommodating portion in a retaining state of the retaining portion. The cassette has a detachment mechanism capable of detaching at least the first bag.

Since the blood bag system includes the cassette, the first bag and the second bag retained by the cassette can be integrally handled. That is, a user can smoothly perform an operation of disposing the first and second bags in the bag accommodating portion by gripping and operating the cassette when setting the blood bag system in the centrifugal transport device. Further, the blood bag system allows the first bag to be detached from the cassette, thereby speeding up the work after removing the blood bag system from the centrifugal transport device. Therefore, the blood bag system can significantly improve the work efficiency.

### Brief Description of Drawings

Fig. 1 is an explanatory view illustrating an overall configuration of a blood bag system and a centrifugal transport device according to an embodiment of the present invention.
Fig. 2 is a plan view illustrating a unit set area of the centrifugal transport device.
Fig. 3 is a front view illustrating a PPP bag, a liquid drug bag, and a cassette.
Fig. 4 is a perspective view illustrating an operation of setting the PPP bag, the liquid drug bag, and the cassette in the centrifugal transport device.
Fig. 5 is a perspective view illustrating an operation of detaching the PPP bag from the cassette.
Fig. 6 is a perspective view schematically illustrating a state where the cassette is folded and set in the blood collection device.
Fig. 7 is a front view illustrating a state when a liquid drug is supplied from the liquid drug bag to a blood bag.

### Description of Embodiments

Hereinafter, preferred embodiments of the present invention will be described in detail with reference to the accompanying drawings.

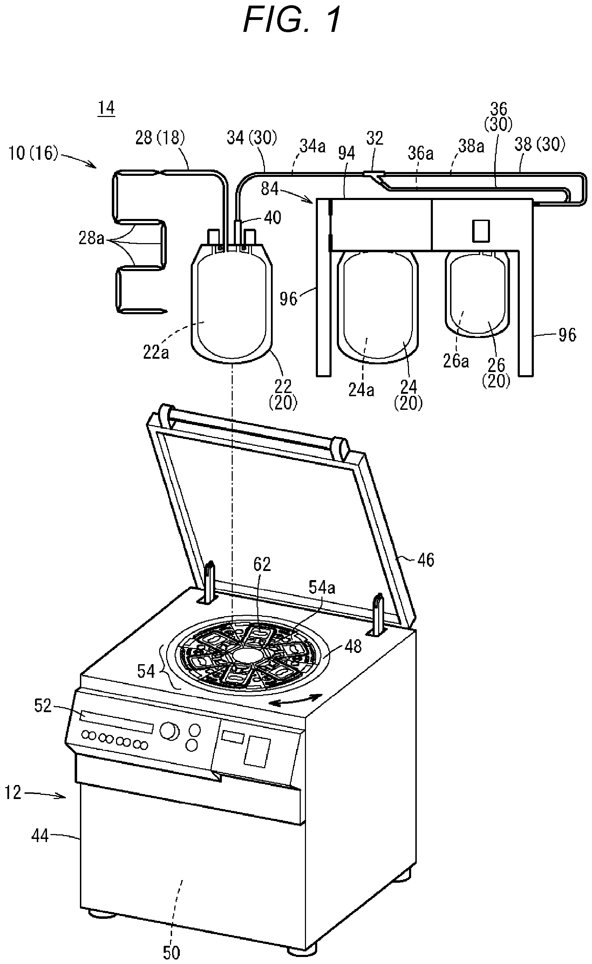

A blood bag system 10 according to an embodiment of the present invention is set in a centrifugal transport device 12 as illustrated in Fig. 1 by a user such as a medical worker. The centrifugal transport device 12 centrifuges blood in the blood bag system 10 to generate and store a plurality of types of blood components. Hereinafter, a configuration including the blood bag system 10 and the centrifugal transport device 12 is referred to as a blood product manufacturing system 14.

The blood bag system 10 includes a pretreatment unit (not illustrated) used for blood sampling before centrifugation and a separator unit 16 that generates blood components by centrifugation and individually stores the blood components, and the both units are connected by a relay tube 18. The separator unit 16 is set in the centrifugal transport device 12 in a state where the relay tube 18 connected to the pretreatment unit is cut into the state illustrated in Fig. 1 before the centrifugation.

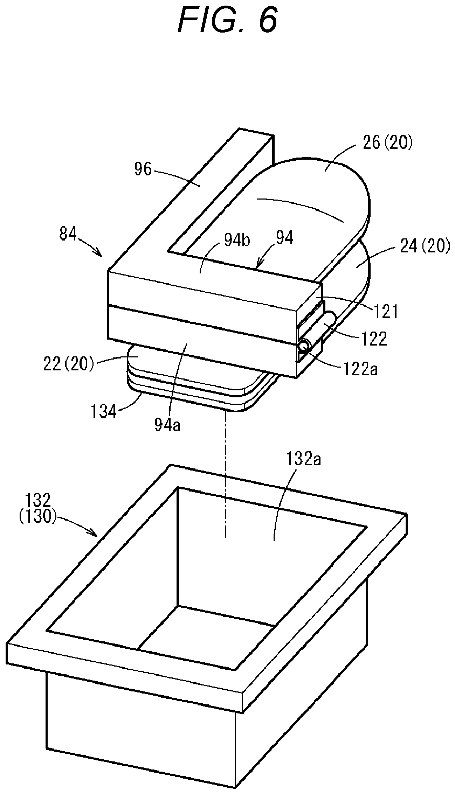

The pretreatment unit of the blood bag system 10 collects whole blood from a donor (blood donor) and removes a predetermined blood component (for example, a white blood cell) from the whole blood. Therefore, the pretreatment unit includes a blood collection needle, a blood collection bag 134 (see Fig. 6), an initial flow blood bag, a filter (for example, a white blood cell removal filter), and the like, and is configured by connecting the respective members via a plurality of tubes. The separator unit 16 is connected to the downstream side of the filter via the relay tube 18. Specifically, the pretreatment unit stores the first blood out of the whole blood of the donor, collected through the blood collection needle, in the initial flow blood bag, and then stores the remaining blood in the blood collection bag 134. Further, the pretreatment unit removes white blood cells in the filter by causing the whole blood, stored in the blood collection bag 134, to flow to the filter, and transports the removed blood (white-blood-cell-removed blood) to the separator unit 16.

On the other hand, the separator unit 16 of the blood bag system 10 includes a plurality of bags 20 (a blood bag 22, a PPP bag 24, and a liquid drug bag 26), and is configured by connecting the respective bags 20 via a plurality of tubes 30.

The blood bag 22 is directly connected to the relay tube 18 connected to the pretreatment unit in a state of the blood bag system 10 provided as a product. Therefore, the blood bag 22 stores the removed blood transported from the filter at the time of collecting blood. The blood bag 22 is set in the centrifugal transport device 12, and a centrifugal force is applied by the operation of the centrifugal transport device 12. As a result, the removed blood in the blood bag 22 is centrifuged into blood components (platelet poor plasma (PPP), red blood cells (RBC)) and the like having different specific gravities. Then, the blood bag 22 transports PPP under the operation of the centrifugal transport device 12 after centrifugation to store only the remaining RBC.

The PPP bag 24 is a medical bag (plasma bag: first bag) that stores PPP (plasma component) in a storage space 24a by receiving the PPP supplied from the blood bag 22. On the other hand, the liquid drug bag 26 is a medical bag (second bag) in which a red blood cell preservation solution (liquid drug), such as a MAP solution, an SAGM solution, and OPTISOL, is stored in advance in a storage space 26a.

In addition, a segment tube 28 is formed before the separator unit 16 is set in the centrifugal transport device 12. The segment tube 28 has a plurality of sealed tubes 28a in which blood of the donor (removed blood) exists. The plurality of sealed tubes 28a are formed so as to be continuous in series by cutting the relay tube 18, which connects the blood bag 22 and the pretreatment unit (filter), near the filter and further sealing the cut tube at predetermined intervals. The segment tube 28 is cut by a user as necessary, and is used for checking blood or the like.

The plurality of tubes 30 of the separator unit 16 branch into a plurality of tubes via a branching connector 32 (Y-type connector). Specifically, the plurality of tubes 30 include a first tube 34 connecting the blood bag 22 and the branching connector 32, a second tube 36 (first tubular body) connecting the PPP bag 24 and the branching connector 32, and a third tube 38 (second tubular body) connecting the liquid drug bag 26 and the branching connector 32. The first tube 34 has a first flow path 34a, the second tube 36 has a second flow path 36a, and the third tube 38 has a third flow path 38a. The first to third flow paths 34a, 36a, and 38a communicate with each other via a flow path in the branching connector 32.

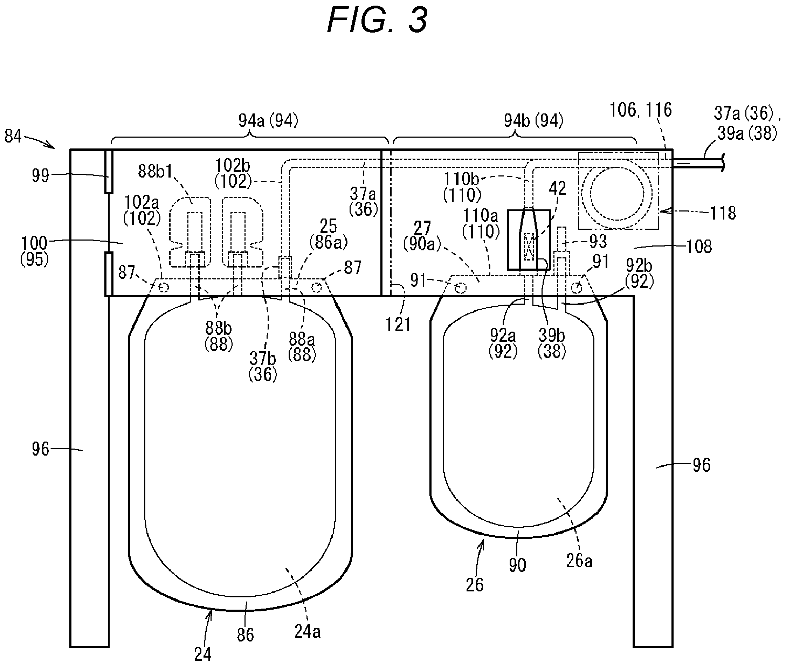

A breakable sealing member 40 (click tip) is provided at an end portion of the first tube 34 on the blood bag 22 side. Similarly, a breakable sealing member 42 (see Fig. 3) is provided at an end portion of the third tube 38 on the liquid drug bag 26 side. The sealing members 40 and 42 block the first flow path 34a and the third flow path 38a until a breaking operation is performed, and prevent a liquid in the blood bag 22 and the liquid drug bag 26 from being transported to the other bag 20.

On the other hand, the centrifugal transport device 12 in which the separator unit 16 of the blood bag system 10 is set includes a box-shaped base body 44, a lid 46 capable of opening and closing an upper surface of the base body 44, and a centrifugal drum 48 provided on the base body 44. In addition, the base body 44 of the centrifugal transport device 12 is provided with a motor (not illustrated) that rotates the centrifugal drum 48, a control unit 50 that controls the operation of the centrifugal transport device 12, and an operation display unit 52 configured to allow the user to perform confirmation and operation.

The centrifugal drum 48 has a plurality of (six) unit set areas 54 in which the separator unit 16 can be set. A height of one unit set area 54 is longer than a longitudinal length of the bag 20, and is set in a range of 60° with respect to a rotation center of the centrifugal drum 48. That is, the six unit set areas 54 are arrayed without a gap along the circumferential direction to form the centrifugal drum 48 which is an annular structure.

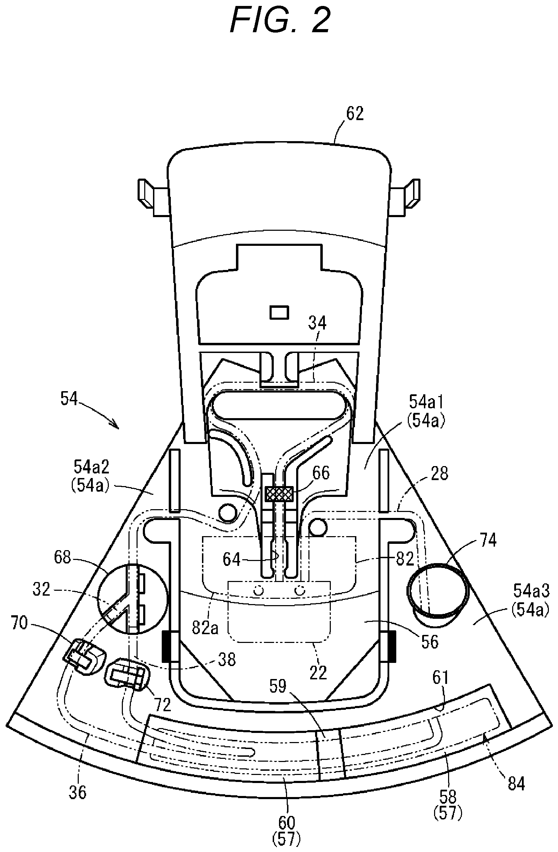

As illustrated in Figs. 1 and 2, the unit set area 54 applies the centrifugal force to the blood bag 22 as the centrifugal drum 48 rotates. Specifically, the unit set area 54 includes a blood bag pocket 56 accommodating the blood bag 22, a PPP bag pocket 58 accommodating a PPP bag 24, and a liquid drug bag pocket 60 accommodating the liquid drug bag 26 at radially outer positions of the centrifugal drum 48.

The blood bag pocket 56 is provided at the circumferential center of the unit set area 54 and has a larger volume than the PPP bag pocket 58 or the liquid drug bag pocket 60. The PPP bag pocket 58 and the liquid drug bag pocket 60 form pockets 57 (bag accommodating portions) of a cassette 84 provided side by side (adjacent) in the circumferential direction on the radially outer side of the blood bag pocket 56. Opening sides of the PPP bag pocket 58 and the liquid drug bag pocket 60 form an arc-shaped common space 61 continuous with each other, and the PPP bag pocket 58 and the liquid drug bag pocket 60 are individually divided with a boundary wall 59 interposed therebetween on the back side of the common space 61.

In addition, an upper surface 54a of the unit set area 54 is configured such that the plurality of tubes 30 of the blood bag system 10 are disposed and retained thereon. The first tube 34 and a part of the segment tube 28 are disposed in a central region 54a1 (first region) on the radially inner side of the blood bag pocket 56 on the upper surface 54a. The central region 54a1 is provided with a lid body 62 that opens and closes an opening of the blood bag pocket 56. Further, a sensor 66 (optical sensor), which detects a part of a breaking portion 64 that breaks the sealing member 40 and a state of blood flowing through the first tube 34, is provided at an arrangement position of the first tube 34 closed by the lid body 62.

A part of the first tube 34 passing through the central region 54a1, the branching connector 32, a part of the second tube 36, and a part of the third tube 38 are disposed in a left region 54a2 (second region) of the upper surface 54a. The left region 54a2 is provided with a holder 68 that retains the branching connector 32, a PPP clamp 70 that opens and closes the second flow path 36a of the second tube 36, and a liquid drug clamp 72 that opens and closes the third flow path 38a of the third tube 38.

A part of the segment tube 28 passing through the central region 54a1 is disposed in a right region 54a3 (third region) of the upper surface 54a. The right region 54a3 is provided with a segment pocket 74 that accommodates the plurality of sealed tubes 28a of the segment tube 28.

In addition, the unit set area 54 includes a slider 82 (pusher), which presses the blood bag 22 after centrifugation, on the radially inner side of the blood bag pocket 56. A surface of the slider 82 facing the blood bag 22 is formed on an inclined surface 82a. The slider 82 moves forward and backward along the radial direction of the centrifugal drum 48 under the control of the control unit 50 (see Fig. 1).

In the unit set area 54, the user accommodates the blood bag 22 storing the removed blood, the empty PPP bag 24, and the liquid drug bag 26 storing the liquid drug in the blood bag pocket 56, the PPP bag pocket 58, and the liquid drug bag pocket 60, respectively. Then, the unit set area 54 centrifuges the removed blood in the blood bag 22 by the rotation of the centrifugal drum 48, advances the slider 82 after the centrifugation to press the blood bag 22. As a result, PPP generated by the centrifugation in the blood bag 22 is transported to the PPP bag 24, and RBC remains in the blood bag 22 after the transport of PPP. Thereafter, the blood bag system 10 is taken out from the centrifugal transport device 12 by the user, and the liquid drug bag 26 is hung on a stand (not illustrated). As a result, the red blood cell preservation solution is supplied from the liquid drug bag 26 to the blood bag 22, and the blood bag 22 is turned into a state of storing the RBC (blood products) containing a liquid drug.

Next, the PPP bag 24, the liquid drug bag 26, and the like of the blood bag system 10 according to the present embodiment will be described with reference to Figs. 3 to 5.

The blood bag system 10 includes the cassette 84 that retains the PPP bag 24 and the liquid drug bag 26 together. The cassette 84 is set in the centrifugal transport device 12 in the state of retaining the PPP bag 24 and the liquid drug bag 26, causes the PPP bag 24 to be accommodated in the PPP bag pocket 58, and causes the liquid drug bag 26 to be accommodated in the liquid drug bag pocket 60.

The cassette 84 locks each of an upper end portion 25 of the PPP bag 24 and an upper end portion 27 of the liquid drug bag 26 to suspend and retain the PPP bag 24 and the liquid drug bag 26. One end of the second tube 36 is connected to the upper end portion 25 of the PPP bag 24, and the cassette 84 retains a predetermined range on one end side of the second tube 36 together. Similarly, one end of the third tube 38 is connected to the upper end portion 27 of the liquid drug bag 26, and the cassette 84 retains a predetermined range on one end side of the third tube 38 together.

The PPP bag 24 and the liquid drug bag 26 are formed in a bag shape by overlapping a plurality of resin sheets and sealing the periphery thereof. A material forming the resin sheet is not particularly limited, and examples thereof include polyolefins such as polyvinyl chloride, an ethylene-vinyl acetate copolymer, polyethylene, and polypropylene. In addition, materials forming the second and third tubes 36 and 38 are not particularly limited, either, and examples thereof include a fluorine-based resin such as polytetrafluoroethylene (PTFE), an ethylene-tetrafluoroethylene copolymer (ETFE), and a perfluoroalkoxy fluorine resin (PFA), an olefin-based resin such as polyethylene and polypropylene or a mixture thereof, polyurethane, polyester, polyamide, a polyether nylon resin, a mixture of the olefin-based resin and an ethylene-vinyl acetate copolymer, and the like.

More specifically, the PPP bag 24 has a seal part 86 surrounding the storage space 24a, and is formed in a rectangular shape in a front view. A pair of retaining holes 87 is provided at both ends in the width direction of a short side seal 86a, which forms the upper end portion 25, in the seal part 86.

In addition, a plurality of ports 88 are provided at the upper end portion 25 of the PPP bag 24. The plurality of ports 88 include a connection port 88a to which the second tube 36 is connected and two extraction ports 88b covered with a cover 88b1 that blocks outflow of blood. Each of the ports 88 is welded in the state of communicating with the storage space 24a at the time of forming the seal part 86.

The second tube 36 includes an elongated tube main body 37a forming most of its entire length, and an end structure 37b that is continuous with an end portion of the tube main body 37a and is formed thick to be connected to the connection port 88a.

On the other hand, the liquid drug bag 26 has a seal part 90 surrounding the storage space 26a, and is formed in a rectangular shape slightly smaller than the PPP bag 24 in a plan view. A pair of retaining holes 91 is provided at both ends in the width direction of the short side seal 90a, which forms the upper end portion 27, in the seal part 90.

In addition, a plurality of ports 92 are also provided in the upper end portion 27 of the liquid drug bag 26. The plurality of ports 92 include a connection port 92a to which the third tube 38 is connected and a filling port 92b to which a filling tube 93 is connected. The filling tube 93 is used when the liquid drug is stored in the storage space 26a, and is cut and sealed after being filled with the liquid drug. The plurality of ports 92 are welded in the state of communicating with the storage space 26a at the time of forming the seal part 90.

The third tube 38 includes an elongated tube main body 39a forming most of its entire length, and a cylindrical end structure 39b that is continuous with an end portion of the tube main body 39a and is formed thick to be connected to the connection port 92a. The above-described sealing member 42 is provided inside the end structure 39b.

The cassette 84 includes a retaining portion 94 that collectively retains the PPP bag 24 and the liquid drug bag 26, and a pair of frame bodies 96 protruding downward (in a direction orthogonal to the width direction) from both ends of the retaining portion 94 in the width direction. Incidentally, the retaining portion 94 is preferably configured to be foldable at the center in the width direction (see also Fig. 6). A configuration for folding the retaining portion 94 will be described in detail later.

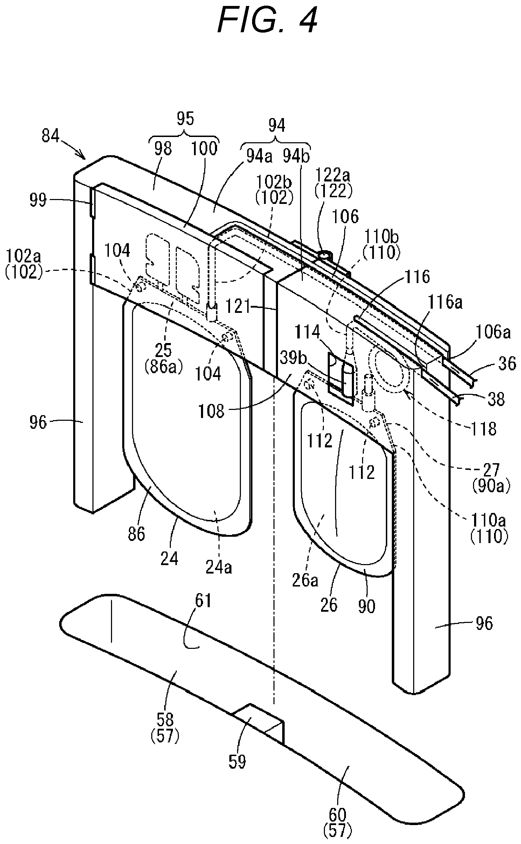

Specifically, as illustrated in Figs. 3 and 4, the retaining portion 94 is formed in a rectangular shape that is long in the lateral direction (left-right direction) and short in the vertical direction (up-down direction) in a front view. In addition, an extending portion of the retaining portion 94 in the longitudinal direction is formed in an arc shape so as to follow a shape of the common space 61 of the unit set area 54 in a plan view. A thickness of the retaining portion 94 in the normal direction (centrifugal direction) is larger than the expansion of the liquid drug bag 26 in which the liquid drug is stored. That is, the PPP bag 24 and the liquid drug bag 26 suspended vertically downward from the retaining portion 94 are not visible in the plan view of the retaining portion 94.

The retaining portion 94 has a PPP bag retaining region 94a on the left side and a liquid drug bag retaining region 94b on the right side when the inner surface side of the arc is viewed. The PPP bag retaining region 94a includes a detachment mechanism 95 that detachably retains the PPP bag 24. On the other hand, the liquid drug bag retaining region 94b retains the liquid drug bag 26 in an undetachable manner.

As illustrated in Figs. 3 to 5, the PPP bag retaining region 94a includes a main body portion 98 that retains the PPP bag 24, and a cover portion 100 attached to an inner surface 98a of the main body portion 98 (on the radially inner side of the arc portion) via a hinge 99. The main body portion 98 is formed to be thicker than the cover portion 100, and a recessed portion 102 (bag retaining portion), which retains the upper end portion 25 of the PPP bag 24 and one end side of the second tube 36, is formed on the inner surface 98a. That is, the detachment mechanism 95 includes the main body portion 98 having the recessed portion 102 and the cover portion 100.

The recessed portion 102 matches shapes of the short side seal 86a of the upper end portion 25 of the PPP bag 24, each of the ports 88, and the second tube 36 (the tube main body 37a and the end structure 37b), and detachably retains the PPP bag 24 and the second tube 36. A bag groove 102a of the recessed portion 102 has a pair of pins 104 to be inserted into the pair of retaining holes 87 while accommodating the upper end portion 25 of the PPP bag 24. The PPP bag 24 is suspended from the PPP bag retaining region 94a as the upper end portion 25 is caught by the pair of pins 104. Incidentally, the retaining portion 94 is not limited to the configuration in which the upper end of the PPP bag 24 is suspended by the pair of pins 104 as described above. For example, the retaining portion 94 may be configured to retain the PPP bag 24 by sandwiching the PPP bag 24 between the main body portion 98 and the cover portion 100 and to release the retaining of the PPP bag 24 by separating the cover portion 100 from the main body portion 98.

A tube groove 102b of the recessed portion 102 is continuous with the bag groove 102a and extends in the up-down direction of the retaining portion 94. The tube groove 102b communicates with a tube open groove 106 (first tubular body groove) formed in an upper end surface of the retaining portion 94.

The tube open groove 106 is open upward, extends short to the radially outer side (in the thickness direction of the retaining portion 94) from the inner surface 98a, then extends in an arc shape on the upper end surface of the retaining portion 94, and communicates with an opening port 106a provided on the side of the liquid drug bag retaining region 94b. That is, the tube open groove 106 extends in both the PPP bag retaining region 94a and the liquid drug bag retaining region 94b, and accommodates the second tube 36 along the extending shape thereof.

The tube open groove 106 has a locking portion (not illustrated) that detachably locks the second tube 36. For example, the locking portion is configured using an inner peripheral surface of the retaining portion 94 forming the tube open groove 106, and the inner peripheral surface is in close contact with an outer peripheral surface of the second tube 36 with an appropriate frictional force.

Meanwhile, the liquid drug bag retaining region 94b is formed in a block 108 continuous with the main body portion 98 of the PPP bag retaining region 94a, and has an arrangement space 110 for retaining the upper end portion 27 of the liquid drug bag 26 and one end side of the third tube 38 therein. The arrangement space 110 matches shapes of the short side seal 90a, each of the ports 92, and the third tube 38 (the tube main body 39a and the end structure 39b) of the upper end portion 27 of the liquid drug bag 26. A bag space 110a of the arrangement space 110 has a pair of pins 112 to be inserted into the pair of retaining holes 91 while accommodating the upper end portion 27 of the liquid drug bag 26. The liquid drug bag 26 is suspended from the liquid drug bag retaining region 94b as the upper end portion 27 is caught by the pair of pins 112.
Incidentally, the liquid drug bag retaining region 94b may be configured to retain the liquid drug bag 26 by sandwiching the liquid drug bag.

A tube space 110b of the arrangement space 110 communicates with the bag space 110a, extends in the upward direction of the bag space 110a, and communicates with a tube open groove 116 (second tubular body groove) formed on the upper end surface of the retaining portion 94. The tube open groove 116 is open upward, extends in an arc shape on the upper end surface of the retaining portion 94, and communicates with an opening port 116a provided on the side of the liquid drug bag retaining region 94b. The third tube 38 is accommodated along the tube space 110b and the tube open groove 116. In the tube space 110b, the end structure 39b of the third tube 38 is disposed in a portion extending in the up-down direction.

The block 108 of the liquid drug bag retaining region 94b has a window portion 114 formed to communicate with the arrangement space 110 and expose the end structure 39b.
The window portion 114 allows the user to access the end structure 39b, and allows implementation of an operation of breaking the sealing member 42 provided in the end structure 39b. Incidentally, a breaking mechanism (not illustrated) for breaking the sealing member 42 may be provided in the window portion 114 or the like of the retaining portion 94.

In addition, the liquid drug bag retaining region 94b includes an accommodating portion 118 that accommodates the third tube 38 formed to be longer than a dimension of the liquid drug bag retaining region 94b in the width direction. For example, the accommodating portion 118 is continuous with the bottom of the tube open groove 116, is formed in a space, formed in the block 108, and accommodates the third tube 38 in a wound state. Incidentally, an accommodation form of the third tube 38 in the accommodating portion 118 is not particularly limited.

The above-described retaining portion 94 causes the PPP bag 24 and the liquid drug bag 26 to be suspended at positions separated from each other by a predetermined interval. This interval is set to be slightly wider than the boundary wall 59 of the centrifugal drum 48. In addition, the retaining portion 94 retains the PPP bag 24 and the liquid drug bag 26 so as not to make contact with the pair of frame bodies 96 on the outer side in the width direction.

In addition, the retaining portion 94 is configured to be foldable in the width direction at a boundary portion 121 between the PPP bag retaining region 94a and the liquid drug bag retaining region 94b as illustrated in Fig. 6 (in Fig. 6, the tube 30 is not illustrated for easy understanding of the drawing). Therefore, the retaining portion 94 has a hinge portion 122 on the radially outer side overlapping the boundary portion 121 (the side opposite to a surface where the cover portion 100 is installed). The hinge portion 122 is capable of relatively rotating the PPP bag retaining region 94a and the liquid drug bag retaining region 94b with a shaft portion 122a extending in the up-down direction (vertical direction) as a base point.

Here, the blood bag system 10 is in a state where the cassette 84 is folded such that the PPP bag 24 and the liquid drug bag 26 overlap each other at the time of collecting blood. Further, the folded cassette 84, the PPP bag 24, and the liquid drug bag 26 are housed in a blister tray 132 of the blood collection device 130 in the state of overlapping the other bags (the blood bag 22, the blood collection bag 134 of the pretreatment unit, the initial flow blood bag, the filter, and the like). The blood collection device 130 applies a negative pressure to all the bags accommodated in an internal space 132a of the blister tray 132 to suck blood (perform vacuum blood collection). That is, since the blood bag system 10 is configured to fold the cassette 84, the blood sampling can be performed by easily accommodating the cassette 84 in the blister tray 132.

Returning to Figs. 3 and 4, the pair of frame bodies 96 of the cassette 84 extends downward from both end portions of the retaining portion 94 in the width direction below the PPP bag 24 and the liquid drug bag 26. A lower end of the frame body 96 makes contact with a bottom wall (not illustrated) of the centrifugal drum 48 forming the PPP bag pocket 58 and the liquid drug bag pocket 60, and defines a set height of the cassette 84 with respect to the centrifugal drum 48.

The blood bag system 10 according to the present embodiment is basically configured as described above, and operations thereof will be described hereinafter.

As described above, the pretreatment unit is used by the user (medical worker) during blood collection in the blood bag system 10. During the blood collection, the user collectively accommodates the plurality of bags in the blister tray 132 of the blood collection device 130 in a state where the cassette 84 is folded and the plurality of bags overlap each other (see Fig. 6). Then, the blood collection device 130 applies the negative pressure to the blister tray 132 to collect blood from the donor. Further, the whole blood of the donor, stored in the blood collection bag 134 of the pretreatment unit, is caused to flow through the filter to obtain removed blood from which a predetermined component (white blood cell) has been removed, and the removed blood is stored in the blood bag 22. Thereafter, the user separates the separator unit 16 of the blood bag system 10 from the pretreatment unit and sets the separator unit 16 in the centrifugal transport device 12 in order to centrifuge the removed blood. The blood bag 22 is accommodated in the blood bag pocket 56 of the unit set area 54.

As illustrated in Fig. 2, the first to third tubes 34, 36, and 38 are disposed in a predetermined path on the upper surface 54a of the unit set area 54. Specifically, the second tube 36 is disposed so as to extend to the outer side in the centrifugal direction from the branching connector 32 in the left region 54a2 of the upper surface 54a and pass through the PPP clamp 70. In addition, the third tube 38 is disposed so as to extend to the outer side in the centrifugal direction from the branch connector 32 in the left region 54a2 of the upper surface 54a and pass through the liquid drug clamp 72, which is similar to the second tube 36.

Then, the user retains the cassette 84 and accommodates the PPP bag 24 and the liquid drug bag 26 together with the cassette 84 in the common space 61 of the unit set area 54 as illustrated in Fig. 4. As described above, the retaining portion 94 matches the arc shape of the common space 61, and the orientation of the cassette 84 is guided such that the arc-shaped inner surface faces the inner side in the centrifugal direction.

When the cassette 84 is moved vertically downward with respect to the common space 61, the PPP bag 24 is inserted into the PPP bag pocket 58, and the liquid drug bag 26 is inserted into the liquid drug bag pocket 60. That is, the cassette 84 can quickly set the PPP bag 24 and the liquid drug bag 26.

In the cassette 84, a lower surface of the retaining portion 94 between the PPP bag 24 and the liquid drug bag 26 just makes contact with the boundary wall 59 in the state where the frame bodies 96 are set in the PPP bag pocket 58 and the liquid drug bag pocket 60. In the set state, the cassette 84 enters the common space 61 up to a lower portion (accommodating portion of the ports 88 and 92 and the like of the PPP bag 24 and the liquid drug bag 26) of the retaining portion 94. On the other hand, the tube open groove 106 of the cassette 84 and a portion extending in the width direction (along the arc) of the tube space 110b protrude above the common space 61. Therefore, the cassette 84 exposes the opening ports 106a and 116a of the retaining portion 94 from the common space 61, and favorably sends the second and third tubes 36 and 38 from the side of the retaining portion 94.

After the blood bag system 10 is set, the centrifugal transport device 12 centrifuges the removed blood in the blood bag 22 into blood components having different specific gravities (PPP, RBC, and the like) by rotating the centrifugal drum 48 under the control of the control unit 50. After such centrifugation, the centrifugal transport device 12 presses the blood bag 22 by the slider 82. As a result, PPP having a light specific gravity flows out from the blood bag 22, and the PPP flows through the first tube 34, the branching connector 32, and the second tube 36 in this order and flows into the PPP bag 24. In addition, air present in the PPP bag 24 or the like during centrifugation or transfer flows out to the second tube 36 according to the extension of the tube open groove 106 on the outer side in the centrifugal direction.

When PPP is transported from the blood bag 22 to the PPP bag 24, the centrifugal transport device 12 retracts the slider 82 so that the blood bag 22 can be taken out from the blood bag pocket 56. Thereafter, the user removes the blood bag system 10 from the centrifugal transport device 12. At this time, the cassette 84 is also taken out, and the PPP bag 24 and the liquid drug bag 26 are quickly separated from the centrifugal transport device 12.

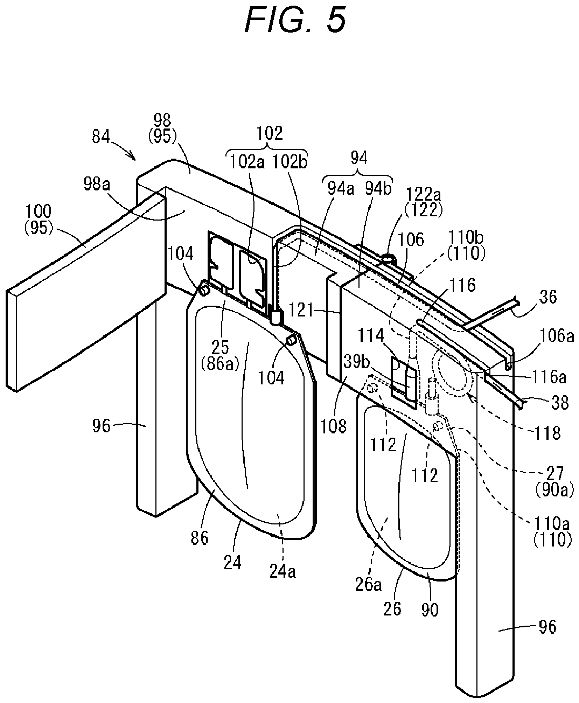

After the take-out, the user performs an operation of separating the PPP bag 24 and the second tube 36 from the cassette 84. In this case, the user opens the cover portion 100 that closes the main body portion 98 to expose the recessed portion 102 as illustrated in Fig. 5. As a result, the PPP bag 24 and the second tube 36 can be taken out from the recessed portion 102. In addition, the second tube 36 can also be taken out from the tube open groove 106, and thus, the PPP bag 24 and the second tube 36 are smoothly separated from the cassette 84.

In addition, the second tube 36 is broken and sealed at an appropriate position by the user, and is further sealed at predetermined intervals in a state where PPP is present inside, thereby being formed into a plurality of sealed tubes (segment tubes) (not illustrated). Each of the sealed tubes is subjected to inspection or the like at the time of using PPP.

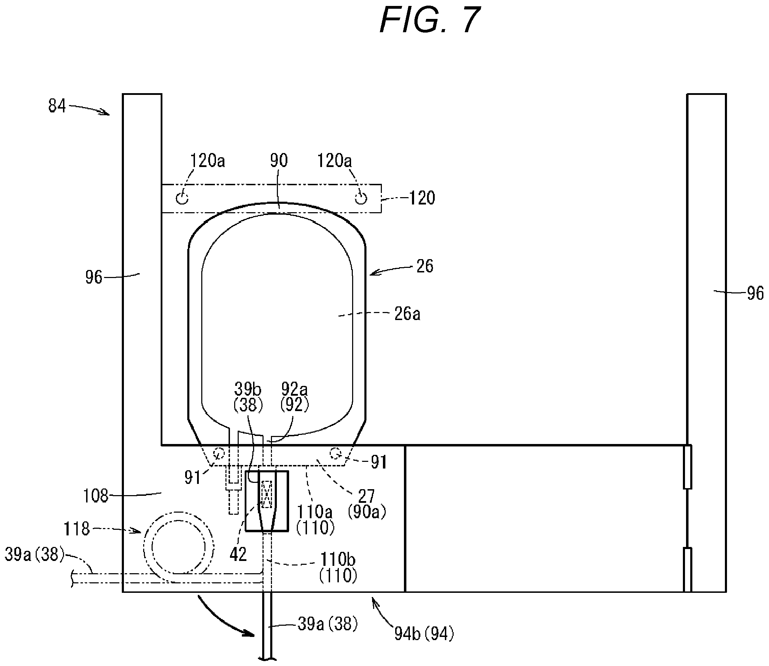

In addition, after the second tube 36 is separated from the cassette 84, the user suspends the liquid drug bag 26 on an addition base to supply the red blood cell preservation solution in the liquid drug bag 26 to the blood bag 22. At this time, the third tube 38 is taken out from the tube open groove 116 to linearly extend along the longitudinal direction of the liquid drug bag 26 as illustrated in Fig. 7. As a result, the red blood cell preservation solution can smoothly flow (under the action of gravity) from the liquid drug bag 26 to the blood bag 22. As a result, RBC containing the red blood cell preservation solution is stored in the blood bag 22.

Incidentally, the blood bag system 10 may have a configuration in which the liquid drug bag 26 and the cassette 84 are collectively suspended from the stand when the liquid drug bag 26 is set on the addition base. For example, as indicated by a two-dot chain line in Fig. 7, the frame body 96 of the cassette 84 may include a sub frame body 120 that retains a part of the liquid drug bag 26 on the side opposite to the retaining portion 94 in a posture with the retaining portion 94 located on the lower side in the gravity direction. The sub frame body 120 has a hooking hole 120a (hooking portion) for hooking the liquid drug bag 26 to the addition base. As a result, it is possible to easily bring the liquid drug bag 26 into the suspended state.

Incidentally, the present invention is not limited to the above-described embodiment, and various modifications can be made in accordance with a gist of the invention. For example, the retaining portion 94 may be configured to cover the entire PPP bag 24 and the entire liquid drug bag 26 in a non-exposed state. In addition, the retaining portion 94 may be configured to suspend and retain the PPP bag 24 connected to the second tube 36 and the liquid drug bag 26 connected to the third tube 38 by fixing the second tube 36 and the third tube 38. Alternatively, the cassette 84 may be configured to set the second tube 36 and the third tube 38 in a free state while retaining the PPP bag 24 and the liquid drug bag 26.

A means for installing the cassette 84 in the common space 61 (the PPP bag pocket 58 and the liquid drug bag pocket 60) is not limited to disposing the pair of frame bodies 96 extending from the retaining portion 94 on the bottom wall. For example, it may be configured such that the pair of frame bodies 96 are formed short to engage the pair of frame bodies 96 with side walls surrounding the common space 61.

In addition, the cover portion 100 is not limited to the configuration of being attached to the main body portion 98 via the hinge 99, and may be, for example, detachably attached to the main body portion 98. Alternatively, the retaining portion 94 may be configured to expose the recessed portion 102 without including the cover portion 100.

Further, the cassette 84 is configured to undetachably retain the liquid drug bag 26 in the present embodiment, but is not limited thereto, and may be also configured to be detachably retain the liquid drug bag 26. As a detachment mechanism (not illustrated) of the liquid drug bag 26, the same mechanism as that of the PPP bag 24 can be adopted.

Furthermore, the centrifugal transport device 12 in the present embodiment includes the PPP bag pocket 58 and the liquid drug bag pocket 60 individually, but is not limited thereto, and the PPP bag 24 and the liquid drug bag 26 may be collectively accommodated in one pocket 57.

Technical ideas and effects that can be grasped from the above-described embodiments are described as follows.

Since the blood bag system 10 includes the cassette 84, the first bag (PPP bag 24) and the second bag (liquid drug bag 26) retained by the cassette 84 can be integrally handled. That is, the user can smoothly perform the work of disposing the PPP bag 24 and the liquid drug bag 26 in the pocket 57 by gripping and operating the cassette 84 when setting the blood bag system 10 in the centrifugal transport device 12. Further, the blood bag system 10 allows the PPP bag 24 to be detached from the cassette 84, thereby speeding up the work after removing the blood bag system 10 from the centrifugal transport device 12. Therefore, the blood bag system 10 can significantly improve the work efficiency.

In addition, the cassette 84 includes the frame bodies 96 which are provided at both the ends of the retaining portion 94 in the width direction and can be disposed on the walls forming the bag accommodating portion (pocket 57). The cassette 84 is smoothly disposed on the wall forming the pocket 57 in the state of retaining the PPP bag 24 and the liquid drug bag 26 by the retaining portion 94 and the frame body 96. Further, the cassette 84 can suppress misalignment of the PPP bag 24 and the liquid drug bag 26 during centrifugation by the centrifugal transport device 12.

In addition, the retaining portion 94 includes the main body portion 98 having the bag retaining portion (recessed portion 102) capable of detachably retaining the first bag (PPP bag 24), and the cover portion 100 capable of maintaining the state where the first bag is retained by the bag retaining portion, thereby forming the detachment mechanism 95. As a result, the user of the blood bag system 10 can operate the cover portion 100 to set the recessed portion 102 in a non-exposed state to prevent detachment of the PPP bag 24, and to set the recessed portion 102 in an exposed state to smoothly detach the PPP bag 24.

In addition, one end of the first tubular body (second tube 36) is connected to the first bag (PPP bag 24), and one end of the second tubular body (third tube 38) is connected to the second bag (liquid drug bag 26). The cassette 84 retains a predetermined range on one end side of the first tubular body and a predetermined range on one end side of the second tubular body together with the first and second bags, and the detachment mechanism 95 is capable of detaching at least the first tubular body. As a result, the cassette 84 can allow the second and third tubes 36 and 38 to be easily disposed at the time of setting the blood bag system 10, and the work efficiency can be further improved.

In addition, the cassette 84 exposes the first tubular body (second tube 36) and the second tubular body (third tube 38) from the same surface of the retaining portion 94, and the detachment mechanism 95 has the first tubular body groove (tube open groove 106) which extends to the opening port 106a formed on one surface of the retaining portion 94 and allows the first tubular body to be taken in and out. As a result, the arrangement of the second and third tubes 36 and 38 are simplified, and it is possible to further suppress the second and third tubes 36 and 38 from projecting. In addition, the cassette 84 can easily separate the second tube 36 from the tube open groove 106.

In addition, the first tubular body groove (tube open groove 106) has a portion that causes the first tubular body (second tube 36) to extend to the outer side in the centrifugal direction of the centrifugal drum 48 from a retaining site of the first bag (PPP bag 24). As a result, the blood bag system 10 can move the air in the PPP bag 24 and the second tube 36 to the outer side in the centrifugal direction during centrifugation of the centrifugal transport device 12, and can reduce the residual of air in the PPP bag 24.

In addition, the cassette 84 has the second tubular body groove (tube open groove 116) which exposes the second bag (liquid drug bag 26) from the retaining portion 94 along the longitudinal direction of the second bag and allows the second tubular body (third tube 38) to be taken in and out, and the second tubular body can extend from a connection site of the second bag along the extension in the longitudinal direction in a state of being taken out from the second tubular body groove. As a result, when the third tube 38 is taken out from the tube open groove 116, the blood bag system 10 can cause the third tube 38 to extend along the extension in the longitudinal direction of the liquid drug bag 26, and cause the liquid drug to smoothly flow from the liquid drug bag 26.

In addition, the retaining portion 94 undetachably retains the second bag (liquid drug bag 26). As a result, the blood bag system 10 can discard the cassette 84 together with the used liquid drug bag 26.

In addition, the retaining portion 94 has the window portion 114 through which the second tubular body (third tube 38) can be partially exposed. As a result, the blood bag system 10 can access the third tube 38 from the outside of the cassette 84 through the window portion 114.

In addition, the breakable sealing member 42 provided on the second tubular body (third tube 38) is located in the window portion 114. As a result, the user can easily break the sealing member 42 provided in the third tube 38.

In addition, the cassette 84 includes the hooking portion (hooking hole 120a) for hooking on a hanging portion (addition base) in the posture with the retaining portion 94 located on the lower side in the direction of gravity with respect to the second bag (liquid drug bag 26). As a result, the liquid drug bag 26 and the cassette 84 can be suspended together, and the liquid drug can smoothly flow out of the liquid drug bag 26.

In addition, the cassette 84 accommodates one side of the first bag (PPP bag 24) formed in a rectangular shape and one side of the second bag (liquid drug bag 26) formed in a rectangular shape therein, thereby retaining the first and second bags in a suspended state. As a result, the structure of the cassette 84 is simplified, and the blood bag system 10 is reduced in weight and can be more easily handled by the user.

In addition, the retaining portion 94 has the hinge portion 122 foldable at the boundary portion 121 between a region for retaining the first bag (PPP bag 24) and a region for retaining the second bag (liquid drug bag 26). As a result, the blood bag system 10 can take a form in which the bags are stacked by folding the cassette 84, for example, and can be easily accommodated in the blister tray 132 of the blood collection device 130 at the time of collecting blood.

## Claims

1. A blood bag system comprising:
a first bag and a second bag which are accommodated adjacent to each other in a bag accommodating portion of a centrifugal drum of a centrifugal transport device; and
a cassette which has a retaining portion that retains both the first bag and the second bag and is accommodated in the bag accommodating portion in a retaining state of the retaining portion,
wherein the cassette has a detachment mechanism capable of detaching at least the first bag.

2. The blood bag system according to claim 1, wherein
the cassette includes frame bodies which are provided at both end portions of the retaining portion in a width direction and are arrangeable on walls forming the bag accommodating portion.

3. The blood bag system according to claim 1 or 2, wherein the retaining portion includes
a main body portion having a bag retaining portion capable of detachably retaining the first bag and
a cover portion capable of maintaining a state of retaining the first bag in the bag retaining portion to form the detachment mechanism.

4. The blood bag system according to any one of claims 1 to 3, wherein
one end of a first tubular body is connected to the first bag,
one end of a second tubular body is connected to the second bag,
the cassette retains a predetermined range on one end side of the first tubular body and a predetermined range on one end side of the second tubular body together with the first and second bags, and
the detachment mechanism is capable of detaching at least the first tubular body.

5. The blood bag system according to claim 4, wherein
the cassette exposes the first tubular body and the second tubular body from one surface of the retaining portion, and
the detachment mechanism includes a first tubular body groove which extends to an opening port formed on the one surface of the retaining portion and allows the first tubular body to be taken in and out.

6. The blood bag system according to claim 5, wherein
the first tubular body groove has a portion that causes the first tubular body to extend to an outer side in a centrifugal direction of the centrifugal drum from a retaining site of the first bag.

7. The blood bag system according to any one of claims 4 to 6, wherein
the cassette includes a second tubular body groove which exposes the second bag from the retaining portion along a longitudinal direction of the second bag and allows the second tubular body to be taken in and out, and
the second tubular body is capable of extending along an extension in the longitudinal direction from a connection site of the second bag in a state of being taken out from the second tubular body groove.

8. The blood bag system according to any one of claims 4 to 7, wherein
the retaining portion undetachably retains the second bag.

9. The blood bag system according to claim 8, wherein
the retaining portion has a window portion capable of partially exposing the second tubular body.

10. The blood bag system according to claim 9, wherein
a breakable sealing member provided on the second tubular body is located in the window portion.

11. The blood bag system according to any one of claims 1 to 10, wherein
the cassette includes a hooking portion for hooking on a hanging portion in a posture with the retaining portion located on a lower side in a direction of gravity with respect to the second bag.

12. The blood bag system according to any one of claims 1 to 11, wherein
the cassette accommodates one side of the first bag formed in a rectangular shape and one side of the second bag formed in a rectangular shape inside to retain the first and second bags in a suspended state.

13. The blood bag system according to any one of claims 1 to 12, wherein
the retaining portion includes a hinge portion foldable at a boundary portion between a region for retaining the first bag and a region for retaining the second bag.
